(19) Europäisches Patentamt
European Patent Office
Office européen des brevets

(11) Publication number: **0 055 996**
**B1**

# (12) EUROPEAN PATENT SPECIFICATION

(45) Date of publication of patent specification: **10.10.84**

(51) Int. Cl.$^3$: **A 61 K 31/70,** A 61 K 31/185

(21) Application number: **82200090.7**

(22) Date of filing: **29.06.81**

(60) Publication number of the earlier application in accordance with Art. 76 EPC: **0 043 274**

## (54) Antithrombotic treatment.

(30) Priority: **22.12.80 US 218413**
**16.06.81 US 271851**

(43) Date of publication of application:
**14.07.82 Bulletin 82/28**

(45) Publication of the grant of the patent:
**10.10.84 Bulletin 84/41**

(84) Designated Contracting States:
**AT BE CH DE FR GB IT LI LU NL SE**

(56) References cited:
**DE-C- 590 580**

**CHEMISTRY & INDUSTRY, no. 40, 4th October 1952, The Society of Chemical Industry London, G.B. C.R. RICKETTS: "Synthetic anticoagulants"**

(73) Proprietor: **T AND R CHEMICALS, INC.**
**700 Celum Road**
**Clint Texas 79836 (US)**

(72) Inventor: **Thompson, Ralph B.**
**35S74 Adams**
**Oak Brook Illinois 60521 (US)**

(74) Representative: **Burford, Anthony Frederick**
**W.H. Beck, Greener & Co. 7 Stone Buildings**
**Lincoln's Inn**
**London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 055 996 B1

## Description

Anticoagulants and antithrombotic agents are a group of compounds with diversified pharmacological actions which are used in a variety of clinical thrombotic disorders. Thrombotic disorders are generally divided into venous thromboses and arterial occlusive disorders. Venous thrombosis of the lower extremities is important, because it can cause pulmonary embolisms which may be fatal. "Heparin" and "Warfarin" are commonly used in clinical medicine for the prevention and treatment of deep venous thromboses and pulmonary embolisms. Their main pharmacological actions are to inhibit or interrupt blood coagulation activity.

Platelets play an important part in arterial thrombosis. Drugs which inhibit platelet aggregation are generally regarded as potentially useful for the prophylactic therapy of arterial thrombotic disorders, including, for example, strokes, myocardial infarctions and peripheral vascular disease. Despite the availability of many agents which possess platelet anti-aggregatory properties, only a few are currently under clinical trial (for example "Aspirin"®, dipyridamole and sulphinpyrazone). None of these agents exhibits unequivocal efficacy. Compounds with more specific pharmacological actions are urgently sought in order to provide better medical care for patients with these serious disorders.

An anticoagulant is a substance which inhibits coagulation of the blood. A platelet anti-aggregatory agent is a substance which inhibits platelet aggregation.

An antithrombotic agent is a substance which inhibits the formation or development of a thrombus (or thrombosis). For present purposes, the term "thrombus" or its equivalent includes the term "embolus", unless otherwise specifically indicated. In general, an antithrombotic agent, in the presence of mammalian blood or appropriately-prepared plasma, may display anticoagulant activity and/or platelet anti-aggregatory activity.

Examples of clinical thrombotic conditions include stroke (as a cerebral vascular thrombosis), myocardial infarction (coronary artery disease), peripheral vascular disease, cardiac valve replacement, deep vein thrombosis and pulmonary embolism.

A class of active agents has now been discovered the members of which, when orally ingested and/or injected, produce amelioration of a thrombotic condition in mammals (including man), when used in anti-thrombotically-effective amounts as described below.

The mechanism(s) by which the active agents function is/are currently unknown; however, an inhibition of platelet aggregation and a prolongation of normal blood coagulation time appear to be associated with their use, in the manner disclosed herein.

Our copending European Patent Application No. 81302950.1 (Publication No. 0043274), from which the present disclosure has been divided, relates to the preparation and use of groups of compounds (and compositions containing them), which are within the class of active compounds in question. One aspect of the invention described and claimed in Application No. 811302950.1 (EP—A—43274) is a pharmaceutical composition which contains at least one carrier substance and, as the active treating agent, at least one compound selected from:

(A) compounds of the formula:

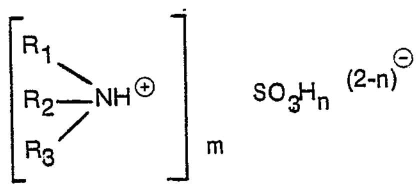

wherein:

$R_1$ is an alkyl radical containing less than 11 carbon atoms, a cycloalkyl radical containing 6 to 10 carbon atoms or a monohydroxyalkyl radical containing less than 11 carbon atoms,

$R_2$ is a hydrogen atom, an alkyl radical containing less than 11 carbon atoms or a monohydroxyalkyl radical containing less than 11 carbon atoms, or

$R_1$ and $R_2$ together form an unsubstituted morpholine, piperidine or hexamethyleneimine ring or such a ring carrying on one of its carbon atoms an alkyl radical containing less than 11 carbon atoms.

$R_3$ is a hydrogen atom, an alkyl radical containing less than 11 carbon atoms, a monohydroxyalkyl radical containing less than 11 carbon atoms or a radical of the formula:

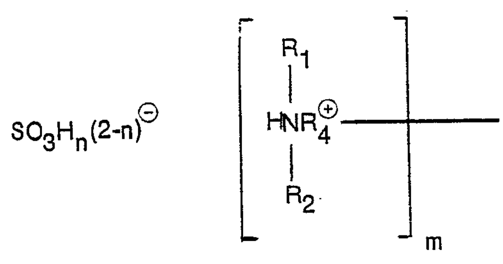

wherein

$R_1$ and $R_2$ have the meanings defined above,

$R_4$ is a divalent saturated aliphatic radical containing less than 11 carbon atoms, and

m is 1 or 2, n is 0 or 1 and the sum of m and n is 2;

compounds of either of the formulae:

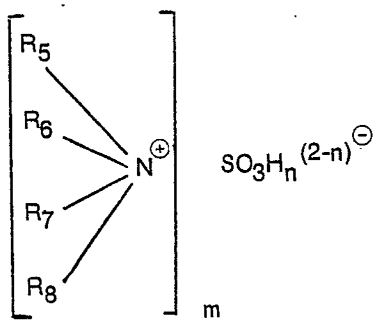

and

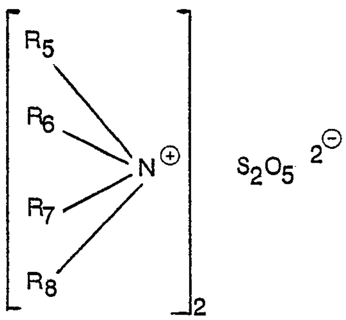

wherein:

$R_5$, $R_6$, $R_7$ and $R_8$ are the same or different and each is an alkyl radical containing less than 11 carbon atoms, and

m and n have the meanings defined above;

(C) compounds of the formula:

$$R—CHOH—SO_3M$$

wherein:

R is a hydrogen atom or a hydroxymethyl radical and

M is an alkali metal or an ammonium group, but excluding:

(a) 0.6M injectable aqueous solutions of ethanolamine bisulphite, and

(b) compositions containing a formaldehyde bisulphite and a protamine.

The present invention concerns other groups of active treating agents within the aforementioned class of active agents.

In accordance with this invention, a pharmaceutical composition for treating an actual or incipient thrombotic condition in a mammal is characterised in that it contains at least one carrier substance and, as the active treating agent, at least one organic compound which has the formula I:

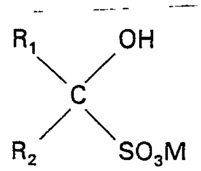

Formula I

wherein:

$R_1$ is a straight-chain hydroxy-substituted alkyl radical containing 3, 4 or 5 hydroxylated carbon atoms,

$R_2$ is a hydrogen atom or a hydroxymethyl radical, and

M is an alkali metal or an ammonium group.

Preferably the active treating agent is sodium glucose bisulphite.

In one aspect, the present invention is concerned with the use of certain bisulphite and sulphite pharmaceutical compositions as antithrombotic, anticoagulant and platelet anti-aggregatory agents in mammalian medicine (including human medicine). These agents are believed to be usable in both arterial and venous thrombotic conditions.

In another aspect, the compositions of the present invention can be used for the control of and/or the prevention of an embolus or a thrombus in man by oral ingestion and/or by injection of a pharmaceutically-effective amount of one or more compounds comprising the active agents of this invention.

In another aspect, the compositions of the present invention can be used for the symptomatic and objective improvement in thrombotic (including cardiovascular) disease conditions, for example, abnormal coagulation or intravascular thrombosis, in man. The term "symptomatic improvement", as used herein, means an improvement in a patient's subjective symptoms (e.g., as reported by the patient). The term "objective improvement", as used herein, means a measurable change in a patient's condition.

More particularly, the compositions of this invention can be used in a process for treating a human or other mammal wherein there is introduced orally and/or by injection into such mammal a pharmaceutically-effective amount of an active agent of this invention as an antithrombotic agent.

Sulphite and/or bisulphite anions do not normally occur in human tissues or blood, so far as is now known.

In medicine, arterial thrombosis is diagnosable, for example, by clinical manifestations, by arteriography and, recently, by an indium 111 platelet labelling technique (see, for example, the article entitled "Differential Effects of Two Doses of Aspirin on Platelet-Vessel Wall Interaction In Vivo" by K. K. Wu et al being published in the Journal of Clinical Investigation, August, 1981).

Also, a thrombosis is detectable, for example, from a patient's conditions symptomatically perceivable by a skilled medical practitioner, and well known to the art of medicine. Objectively, various methods are available, including venography, impedance plethysmography, doppler ultrasound and the $^{125}I$-fibrinogen test (see, for example, the articles by Kakkar, "Archives of Surgery", 104, page 152 (1972) and by Kelton, J. G. et al, Journal of Clinical Investigation, Vol. 62, pgs. 892—895, (1978)).

The present invention does not contemplate feeding a normal patient (that is, one not suffering from a thrombotic condition) an active agent of this invention at a pharmaceutically-effective dosage as indicated herein.

The term "thrombotic condition" as used herein, means both:

(a) an existing thrombus (including an embolus);

(b) an incipient thrombus (including an incipient embolus).

An incipient "thrombus" or "incipient thrombotic condition", as used herein, is a condition which can exist in a patient who is predisposed to the development of a thrombotic condition. For example, diabetes mellitus and hyperlipidemia are conditions which predispose a patient to arterial thrombosis. On the other hand, surgery, trauma and bed rest, for example, predispose a patient to venous thrombosis.

Those skilled in the practice of medicine routinely determine the presence of a thrombotic condition (including an actual thrombus in a patient).

Preferably, the administration of the compositions of this invention *in vivo* involves introducing into the blood of a patient such as a human, the equivalent of 1 to 100 milligrams per kilogram of mammal body weight (including human) per day, though larger or smaller dosage rates may be employed, if desired, within the scope of this invention. The exact amount or dose in any given case is selected to be sufficient and appropriate for achieving a desired antithrombotic effect.

In general, such an introduction may be commenced at a dosage rate within the range above indicated as soon as a thrombotic condition (or a thrombus) is found in a patient.

For example, it is preferred that as a first step, a determination is made that a patient suffers from a thrombotic condition. Then, one starts orally feeding and/or injecting such patient with at least one active agent of the present invention at an effective dosage rate in the range indicated above. Currently, an especially preferred dosage rate is 20 to 50 mg/kg per day. Preferably, at least two or three spaced doses per day are given, each such dose being conveniently administered around a mealtime. Any convenient dosage arrangement can be employed.

Not uncommonly, it is desirable or necessary to start treatment immediately upon the discovery of a patient's thrombotic condition, in order to avoid damage or injury or perhaps even death of the patient, as from an embolus. If oral administration is not convenient or rapid enough, the active agent can be directly introduced by injection into the patient, if desired, such as intravenously, intramuscularly or subcutaneously. When an active agent is directly introduced, it is preferably dissolved in an aqueous medium, the total amount of active agent introduced into such medium preferably being within the range from 1 to 11 weight percent (based on the total solution weight). Distilled water is preferred as the aqueous medium. If desired, conventional (standardized) aqueous media can be used as vehicles for such introduction; for example, standard saline solutions can be used as vehicles.

In order to evaluate an antithrombotic effect, it is preferable to withdraw samples of blood from a patient undergoing treatment and measure platelet aggregation. One method is described by Born in Nature 194, pp, 927—929 (1962) and may be used for this purpose, if desired.

After administration has started, the dosage rate is preferably adjusted to a value which is sufficient to disrupt platelet function and/or coagulation factors and thereby achieve a desired antithrombotic effect.

An active agent of this invention is characteristically capable of exhibiting platelet aggregation both *in vitro* and *in vivo*. Also, such an active agent is characteristically capable of lengthening both PT (propthrombin time) and PTT (blood partial thromboplastin time) *in vitro*. The dosage rate of the active agent is currently believed to be related to the resulting effects upon blood factors, such as inhibition of platelet aggregation. Consequently, under this preferred procedure, use of an active agent at a suitable dose for an individual patient ameliorates that patient's thrombotic condition.

Selected blood parameters of a patient are preferably determined before dosing with an active agent is started, when time permits. Preferably, dosage rate adjustment is made while administration of an active agent is continuing. The amount of adjustment (or incremental change in dosage) is determinable by comparing a patient's measured values during administration of active agent to desired values (such as the patient's own corresponding starting values or normal species, e.g. human, values). Inhibition of platelet aggregation can be used for such measurements. Then, the deviation, if any, from the patient's measured values is compared to the desired values (the patient's starting values or normal species values, for example). Then, a change in dosage rate may be made to correct any deviation so determined.

For instance, in humans, normal values for platelet aggregation are dependent upon the particular agent used for stimulation. For example, when adenosine diphosphate (ADP) at 3 micromolar concentration is employed, platelet aggregation values fall typically in the range from 50% to 100% of light transmission. Other stimulation agents include collagen, epinephrine and arachidonic acid.

Also, in humans, normal PT values fall in the range from 11 to 13 seconds, while normal PTT values fall in the range from 25 to 41 seconds. If PT values and/or PTT values could be measured

in a given patient, for the purpose of achieving a desired antithrombotic effectiveness, it is currently estimated that a lengthening of PTT value to 1.5 to 2 times a PTT value in such normal range in a given starting patient is appropriate or suitable for antithrombotic effectiveness, which amounts to a PTT value for a given patient of 45 to 60 seconds; such an estimate is consistent, for example, with the lengthened PTT values achieved in the human use of heparin, sometimes employed previously as an antithrombotic agent. Similarly, it is currently estimated that a lengthening of PT value to 2.0 times a PT value in such normal range in a given starting patient is appropriate or suitable for antithrombotic effectiveness, which amounts to a PT value for a given patient of 22 to 26 seconds; such an estimate is consistent, for example, with the lengthened PT values achieved in the human use of coumadin (Warfarin), sometimes employed previously as an antithrombotic agent. The active agents of the present invention, contrary to such prior art agents, surprisingly appear to affect both PT and PTT values.

The mechanism by which the present active agents work is apparently substantially different from, and not comparable to, the prior art agents. Study and evaluation of the active agents of this invention continues.

Contrary to prior agents (such as Heparin and coumadin), the active agents of the present invention appear to affect both blood coagulation factors and platelet aggregation. Conveniently and preferably, measurements of blood factors are carried out periodically, such as every 3 to 7 days, on a patient undergoing treatment under the practice of this invention.

An active agent can be given orally, in the form of a capsule or tablet, or in the form of a solution (e.g. aqueous). Also, an active agent can be injected in the form of an aqueous solution.

A particularly preferred antithrombotic field of use is in post-operative treatment, as when arteries or deep veins may be involved in, or threatened by, a thrombotic condition.

By way of explanation, as those familiar with mammalian anatomy appreciate, the venous system in the lower extremities consists of superficial and deep veins. Because of the manner in which the deep veins interconnect and supply blood to the heart and lungs, a thrombus occurring in the deep veins, but not in the superficial veins, can become the source of a blood clot which is moved through the veins and becomes lodged in the lungs, resulting in a pulmonary embolus, which can have obvious catastrophic effects (including causing death). Examples of deep veins include the iliac, the femoral, the popliteal and the calf veins. The prevention of pulmonary emboli following surgery affecting the deep veins in the lower extremities is a significant medical problem. One solution to this problem is to prevent thrombi from occurring and/or developing in deep veins. To achieve this, active agents of this invention appear to be well suited. Thus, by administrating the compositions of this invention, one may achieve a symptomatic and objective improvement of a deep vein thrombotic complication in a patient during post-operative care, inhibiting intravascular thrombus formation (including embolism).

In one preferred administration of the compositions of this invention, an aqueous solution of 1 to 10 percent by weight of an active agent of this invention is prepared. Then, this solution isorally consumed by a human or is injected at a total (or accumulated) dosage rate ranging from 1.0 to 50 mg per kg of body weight per day, more preferably in the form of at least two spaced doses per day, and still more preferably in the form of at least three spaced doses per day, each dose preferably being taken around a meal-time. Instead, solid or encapsulated active agents may be orally consumed.

Because of a tendency for bisulphites to undergo oxidation when in aqueous solution, it is currently preferred to minimize contact of active agents with oxygen before use. However, the compounds of the above-defined formula appear to be stable towards oxygen. It is preferred in practising this invention to employ a solution in which the water used is purified (e.g., filtered, deionized or distilled). After preparation, the solution is preferably stored in a closed container to reduce oxidation.

Such an aqueous solution can be directly used in carrying out this invention, in which case such a solution can be dispensed dropwise or it can be encapsulated, for instance, and used as measured dosage units, as desired.

Symptomatic improvement in varicose veins and in haemorrhoids may be observable when using an active agent of this invention.

Since aqueous solutions of sulphur dioxide display a capacity to lengthen PT and PTT values at least to the extent indicated above, aqueous solutions of sulphur dioxide can be used in combination with, e.g. in admixture with, aqueous solutions of compounds of the formula defined above.

One preferred class of compositions for use in practising the present invention comprise isotonic aqueous solutions of a sugar (such as a 5 weight percent solution of glucose), and additionally dissolved therein, a bisulphite or sulphite (preferably an inorganic bisulphite such as sodium bisulphite), in an amount sufficient to provide a desired therapeutic dose for antithrombotic purposes. Such a composition is useful where an active agent of this invention is being utilized by injection (e.g. intravenously), for example when it is being utilized at a relatively low dosage rate over an extended period of time.

Aqueous solutions of oxygen-sensitive bisulphites with sugar (e.g., glucose) are advantageous, because they are less susceptible to autooxidation than such bisulphites alone in aqueous solution.

Such solutions contain an equilibrium mixture of sugar, bisulphites and the reaction products of such sugar and such bisulphites, which are substituted hydroxy-methanesulphonic acids. As regards the ability of such monosaccharide-containing solutions to affect PT and/or PTT values, it is surprising and unexpected that such solutions do extend PT and PTT values, since investigation has revealed that bisulphite adducts of aliphatic (including cycloaliphatic) and aromatic aldehydes and ketones having more than one carbon atom are substantially without any ability to affect PT and PTT values.

Preferably, such compositions are isotonic (as shown, for example, by an osmolarity machine), because such a solution avoids lysis of red blood cells. A most preferred sugar is glucose. A most preferred bisulphite is sodium bisulphite. The glucose can be present in molar excess relative to the bisulphite.

One preferred aqueous composition is suitable for the treatment of thrombotic conditions selected from (a) deep vein thrombosis, (b) incipient deep vein thrombosis due to surgery, trauma, prolonged bed rest or obstetrics, (c) arterial thrombosis and (d) incipient arterial thrombosis due to diabetes mellitus or hyperlipidemia. Such a composition comprises on a 100 weight % starting material basis:

(A) 4% to 7% of at least one monosaccharide selected from aldoses and 2-carbonyl ketoses containing 5 to 7 carbon atoms,

(B) 1% to 4% of one or more alkali metal or ammonium bisulphites or sulphites and

(C) 89% to 95% of water

A preferred method for treating a thrombotic condition in a mammal (including man), with a composition of the present invention, is characterized by:

(A) determining from a sample of blood removed from the mammal

(1) one or both of the blood coagulation factors consisting of prothrombin time and partial thromboplastin time and

(2) the platelet aggregation factor,

(B) when such factors fall within normal ranges for such mammal, administering to such mammal a pharmaceutical composition of the invention at a dosage rate and for a time sufficient to inhibit the blood coagulation and platelet aggregation factors to an extent which is at least sufficient to inhibit thrombus development in the mammal but insufficient to cause spontaneous internal bleeding,

(C) at intervals during administration, removing further samples of blood from the mammal and determining the inhibited blood coagulation and platelet aggregation factors,

(D) adjusting administration of the composition so as to achieve and maintain predetermined desired values for the lengthened blood coagulation factor(s) and the inhibited platelet aggregation factor in the mammal, and

(E) continuing steps (B), (C) and (D) until the mammal has recovered from the thrombotic condition.

The compositions of the present invention can also be used *in vivo* or *in vitro* for inhibiting blood coagulation factors and for inhibiting platelet aggregation involving adding to blood and/or plasma derived from such blood an active agent of this invention.

The present invention is further illustrated by the following Examples. Reference may also be made to the disclosures, including specific Examples, contained in the aforesaid European Application No. 81302950.1 (EP—A—43274) and also European Application No. 82200091.5 (EP—A—55702) relating respectively to organic and inorganic compounds, the latter having been divided out of the former, like the present disclosure. It is considered that a fuller understanding of the respective inventions can be obtained from the various disclosures when taken in conjunction.

Example A

A solution containing 2% by weight of sodium bisulphite equivalent was prepared by dissolving the desired amount of the bisulphite in one equivalent of glucose.

Example B

The procedure of Example A was repeated, except that four equivalents of glucose were used.

Example C

The procedure of Example B was repeated, except that the product was heated on a steam bath (at about 90°C) for 1 hour.

Example D

The procedure of Example A was repeated, except that fructose was used in place of glucose.

Example E

The procedure of Example A was repeated, except that arabinose was used in place of glucose.

Example 1

*In vitro* assays of the product of each of Examples A to C and certain of the Examples included in European Application EP—A—43274 were conducted for coagulation factors as described in "Human Blood Coagulation, Haemostasis and Thrombosis", edited by Rosemary Biggs, published by Blackwell Scientific Publications, Oxford, England (2nd Edition), pages 670—705, 1976. The results are shown in the Table below.

As is apparent from the foregoing specification, the present invention can be carried out with a wide variety of alterations and modifications. For this reason, it is to be fully understood that all of the foregoing is intended to be merely illustrative and is not to be taken as restrictive or otherwise limiting of the present invention, excepting as defined in the appended claims.

TABLE

CARBONYL ADDUCTS

| | PT(sec) (1) REPLICATIONS | | | PTT(sec) (1) REPLICATIONS | | |
|---|---|---|---|---|---|---|
| | I | II | III | I | II | III |
| $NaHSO_3$ in 1/1 glucose | 20.9 | 20.3 | 19.9 | 65.5 | 63.9 | 65.8 |
| $NaHSO_3$ in 1/4 glucose | 21.3 | 21.9 | 21.5 | 68.5 | 69.0 | 70.2 |
| $NaHSO_3$ in 1/4 glucose heated | 22.8 | 21.5 | 21.0 | 77.2 | 70.4 | 75.6 |
| $NaHSO_3$ in water | 20.2 | 20.3 | 20.0 | 64.4 | 63.2 | 63.9 |
| Control | 13.1 | 13.1 | 13.0 | 38.2 | 38.3 | 39.2 |
| Sodium formaldehyde bisulphite | 20.5 | | | 63.5 | | |
| Control | 14.1 | | | 50.5 | | |

(1) Used at 0.01 Molar Concentration Based on $NaHSO_3$

**Claims for the Contracting States: BE CH DE FR GB IT LI LU NL SE**

1. A pharmaceutical composition for treating an actual or incipient thrombotic condition in a mammal, characterized in that it contains at least one carrier substance and, as the active treating agent, at least one organic compound which has the formula:

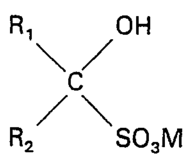

wherein:

$R_1$ is a straight-chain hydroxy-substituted alkyl radical containing 3, 4 or 5 hydroxylated carbon atoms,

$R_2$ is a hydrogen atom or a hydroxymethyl radical, and

M is an alkali metal or an ammonium group.

2. A pharmaceutical composition as claimed in Claim 1, wherein the active treating agent is a bisulphite adduct of an aldose of 5 to 7 carbon atoms.

3. A pharmaceutical composition as claimed in Claim 2, wherein the aldose is arabinose or glucose.

4. A pharmaceutical composition as claimed in Claim 3, wherein the active treating agent is sodium glucose bisulphite.

5. A pharmaceutical composition as claimed in Claim 1, wherein the active treating agent is a bisulphite adduct of a 2-carbonyl ketose of 5 to 7 carbon atoms.

6. A pharmaceutical composition as claimed in Claim 5, wherein the ketose is fructose.

7. A pharmaceutical composition as claimed in any one of the preceding Claims, wherein M is sodium.

8. A pharmaceutical composition as claimed in any one of the preceding Claims, wherein the composition is in an orally administrable form.

9. A pharmaceutical composition as claimed in Claim 8, wherein the composition is an aqueous solution containing 1 to 10% by weight of the active treating agent.

10. A pharmaceutical composition as claimed in any one of Claims 1 to 7, wherein the composition is in an injectable form.

11. A pharmaceutical composition as claimed in claim 10, wherein the composition is an injectable aqueous solution containing 1 to 11% by weight of the active treating agent.

12. A pharmaceutical composition as claimed in any one of the preceding Claims, wherein the active treating agent is the only pharmacologically active agent in the composition.

13. A pharmaceutical composition as claimed in any one of Claims 1 to 11 wherein the composition is in the form of an isotonic aqueous solution of a sugar having dissolved therein an alkali metal or ammonium bisulphite or sulphite whereby the solution contains an equilibrium mixture of sugar, bisulphite or sulphite, and bisulphite adduct of the sugar.

14. A pharmaceutical composition as claimed in Claim 13, comprising, by weight,

(A) 4% to 7% of at least one monosaccharide selected from aldoses and 2-carbonyl ketoses containing 5 to 7 carbon atoms,

(B) 1% to 4% of one or more alkali metal or ammonium bisulphites or sulphites and

(C) 89% to 95% of water.

15. A compound as defined in any one of Claims 1 to 7, for use in treating an actual or incipient thrombotic condition in a human or other mammal.

**Claims for the Contracting State: AT**

1. A method of preparing pharmaceutical composition for treating an actual or incipient thrombotic condition in a mammal, characterized in that it comprises mixing at least one carrier substance with, as the active treating agent, at least one organic compound which has the formula:

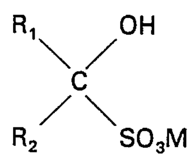

wherein:

$R_1$ is a straight-chain hydroxy-substituted alkyl radical containing 3, 4 or 5 hydroxylated carbon atoms,

$R_2$ is a hydrogen atom or a hydroxymethyl radical, and

M is an alkali metal or an ammonium group.

2. A method as claimed in Claim 1, wherein the active treating agent is a bisulphite adduct of an aldose of 5 to 7 carbon atoms.

3. A method as claimed in Claim 2, wherein the aldose is arabinose or glucose.

4. A method as claimed in Claim 3, wherein the active treating agent is sodium glucose bisulphite.

5. A method as claimed in Claim 1, wherein the active treating agent is a bisulphite adduct of a 2-carbonyl ketose of 5 to 7 carbon atoms.

6. A method as claimed in Claim 5, wherein the ketose is fructose.

7. A method as claimed in any one of the preceding claims, wherein M is sodium.

8. A method as claimed in any one of the preceding claims, wherein the composition is in an orally administrable form.

9. A method as claimed in Claim 8, wherein the composition is an aqueous solution containing 1 to 10% by weight of the active treating agent.

10. A method as claimed in any one of Claims 1 to 7, wherein the composition is in an injectable form.

11. A method as claimed in Claim 10, wherein the composition is an injectable aqueous solution containing 1 to 11% by weight of the active treating agent.

12. A method as claimed in any one of the preceding claims, wherein the active treating agent is the only pharmacologically active agent in the composition.

13. A method as claimed in any one of Claims 1 to 11 wherein the composition is in the form of an isotonic aqueous solution of a sugar having dissolved therein an alkali metal or ammonium bisulphite or sulphite whereby the solution contains an equilibrium mixture of sugar, bisulphite or sulphite, and bisulphite adduct of the sugar.

14. A method as claimed in Claim 13, characterised in that the composition comprises, by weight,

(A) 4% to 7% of at least one monosaccharide selected from aldoses and 2-carbonyl ketoses containing 5 to 7 carbon atoms,

(B) 1% to 4% of one or more alkali metal or ammonium bisulphites or sulphites and

(C) 89% to 95% of water.

**Revendications pour les Etats contractants: BE CH LI DE FR GB IT LU NL SE**

1. Composition pharmaceutique pour le traitement d'un état thrombosique établi ou débutant chez un mammifère, caractérisée en ce qu'elle contient au moins une substance vectrice et, en tant qu'agent de traitement actif, au moins un composé organique qui répond à la formule

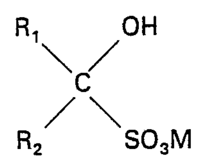

dans laquelle:

$R_1$ est un radical alcoyle en chaîne droite substitué par hydroxyle, contenant 3, 4 ou 5 atomes de carbone hydroxylés,

$R_2$ est un atome d'hydrogène ou un radical hydroxyméthyle et

M est un métal alcalin ou un groupe ammonium.

2. Composition pharmaceutique selon la revendication 1, caractérisée en ce que l'agent de traitement actif est un produit d'addition bisulfitique, d'un aldose contenant 5 à 7 atomes de carbone.

3. Composition pharmaceutique selon la revendication 2, caractérisée en ce que l'aldose est l'arabinose ou le glucose.

4. Composition pharmaceutique selon la revendication 3, caractérisée en ce que l'agent de traitement actif est le bisulfite de glucose sodique.

5. Composition pharmaceutique selon la revendication 1, caractérisée en ce que l'agent de traitement actif est un produit d'addition bisulfitique d'un cétose 2-carbonylé contenant 5 à 7 atomes de carbone.

6. Composition pharmaceutique selon la revendication 5, caractérisée en ce que le cétose est le fructose.

7. Composition pharmaceutique selon l'une quelconque des revendications 1 à 6, caractérisé en ce que M est le sodium.

8. Composition pharmaceutique selon l'une quelconque des revendications 1 à 7, caractérisé en ce qu'elle se présente sous forme administrable par voie orale.

9. Composition pharmaceutique selon la revendication 8, caractérisée en ce que la composition est une solution aqueuse contenant 1 à 10% en poids de l'agent de traitement actif.

10. Composition pharmaceutique selon l'une quelconque des revendications 1 à 7, caractérisée en ce qu'elle se présente sous une forme injectable.

11. Composition pharmaceutique selon la revendication 10, caractérisée en ce que la composition est une solution aqueuse injectable contenant 1 à 11% en poids de l'agent de traitement actif.

12. Composition pharmaceutique selon l'une quelconque des revendications 1 à 11, caractérisée en ce que l'agent de traitement actif est le seul agent pharmacologiquement actif contenu dans la composition.

13. Composition pharmaceutique selon l'une quelconque des revendications 1 à 11, caractérisée en ce qu'elle se présente sous la forme d'une solution aqueuse isotonique d'un sucre, dans laquelle est dissous un bisulfite ou sulfite de métal alcalin ou d'ammonium, ce qui fait que la solution contient un mélange en équilibre de sucre, de bisulfite ou de sulfite et de produit à addition bisulfitique du sucre.

14. Composition pharmaceutique selon la revendication 13, caractérisée en ce qu'elle comprend en poids:

A) 4% à 7% d'au moins un monosaccharide choisi parmi des aldoses et des cétoses 2-carbonylés contenant 5 à 7 atomes de carbone,

B) 1% à 4% d'un ou plusieurs bisulfites ou sulfites d'ammonium ou de métal alcalin,

C) 89% à 95% d'eau.

15. Composé tel que défini dans l'une quelconque des revendications 1 à 7, utilisable dans le traitement d'un état thrombosique établi ou débutant chez l'homme ou d'autres mammifères.

**Revendications pour l'Etat contractant: AT**

1. Procédé de préparation d'une composition pharmaceutique pour le traitement d'un état thrombosique établi ou débutant chez un mammifère, caractérisé en ce qu'il consiste à mélanger au moins une substance vectrice avec, en tant qu'agent de traitement actif, au moins un composé organique qui répond à la formule

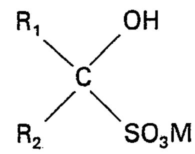

dans laquelle:

$R_1$ est un radical alcoyle en chaîne droite substitué par hydroxyle, contenant 3, 4 ou 5 atomes de carbone hydroxylés,

$R_2$ est un atome d'hydrogène ou un radical hydroxyméthyle et

M est un métal alcalin ou un groupe ammonium.

2. Procédé selon la revendication 1, caractérisé en ce que l'agent de traitement actif est un produit d'addition biosulfitique d'un aldose contenant 5 à 7 atomes de carbone.

3. Procédé selon la revendication 2, caractérisé en ce que l'aldose est l'arabinose ou le glucose.

4. Procédé selon la revendication 3, caractérisé en ce que l'agent de traitement actif est le bisulfite de glucose sodique.

5. Procédé selon la revendication 1, caractérisé en ce que l'agent de traitement actif est un produit d'addition bisulfitique d'un cétose 2-carbonylé contenant 5 à 7 atomes de carbone.

6. Procédé selon la revendication 5, caractérisé en ce que le cétose est le fructose.

7. Procédé selon l'une quelconque des revendications 1 à 6, caractérisé en ce que M est le sodium.

8. Procédé selon l'une quelconque des revendications 1 à 7, caractérisé en ce que la composition se présente sous une forme administrable par voie orale.

9. Procédé selon la revendication 8, caractérisé en ce que la composition est une solution aqueuse contenant 1 à 10% en poids de l'agent de traitement actif.

10. Procédé selon l'une quelconque des revendications 1 à 7, caractérisé en ce que la composition se présente sous une forme injectable.

11. Procédé selon la revendication 10, caractérise en ce que la composition est une solution aqueuse injectable, contenant 1 à 11% en poids de l'agent de traitement actif.

12. Procédé selon l'une quelconque des revendications 1 à 11, caractérisé en ce que l'agent de traitement actif est le seul agent pharmacologiquement actif contenu dans la composition.

13. Procédé selon l'une quelconque des revendications 1 à 11, caractérisé en ce que la composition se présente sous la forme d'une solution aqueuse isotonique d'un sucre, dans laquelle est dissous un bisulfite ou sulfite de métal alcalin ou d'ammonium, de telle manière que la solution contienne un mélange en

équilibre de sucre, de bisulfite ou de sulfite et de produit d'addition bisulfitique du sucre.

14. Procédé selon la revendication 13, caractérisé en ce que la composition comprend, en poids:

A) 4% à 7% d'au moins un monosaccharide choisi parmi des aldoses et des cétoses 2-carbonylés contenant 5 à 7 atomes de carbone,

B) 1% à 4% d'un ou de plusieurs bisulfites ou sulfites d'ammonium ou de métal alcalin,

C) 89% à 95% d'eau.

**Patentansprüche für die Vertragsstaaten: BE CH LI DE FR GB IT LU NL SE**

1. Pharmazeutische Zusammensetzung zur Behandlung einer akuten oder beginnenden Thrombose bei Säugern, dadurch gekennzeichnet, daß sie mindestens eine Trägersubstanz und als aktives Behandlungsmittel mindestens eine organische Verbindung mit der Formel

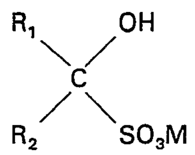

enthält, in der $R_1$ ein geradkettiger, hydroxysubstituierter Alkylrest mit 3,4 oder 5 hydroxylierten Kohlenstoffatomen ist, $R_2$ ein Wasserstoffatom oder ein Hydroxymethylrest ist und M ein Alkalimetall oder eine Ammoniumgruppe ist.

2. Pharmazeutische Zusammensetzung nach Anspruch 1, dadurch gekennzeichnet, daß das aktive Behandlungsmittel ein Bisulfit-addukt einer Aldose mit 5 bis 7 Kohlenstoffatomen ist.

3. Pharmazeutische Zusammensetzung nach Anspruch 2, dadurch gekennzeichnet, daß die Aldose Arabinose or Glucose ist.

4. Pharmazeutische Zusammensetzung nach Anspruch 3, dadurch gekennzeichnet, daß das aktive Behandlungsmittel Natriumglucosebisulfit ist.

5. Pharmazeutische Zusammensetzung nach Anspruch 1, dadurch gekennzeichnet, daß das aktive Behandlungsmittel ein Bisulfitaddukt einer 2-Carbonylketose mit 5 bis 7 Kohlenstoffatomen ist.

6. Pharmazeutische Zusammensetzung nach Anspruch 5, dadurch gekennzeichnet, daß die Ketose Fructose ist.

7. Pharmazeutische Zusammensetzung nach einem der vorangehenden Ansprüche, dadurch gekennzeichnet, daß M Natrium ist.

8. Pharmazeutische Zusammensetzung nach einem der vorangehenden Ansprüche, dadurch gekennzeichnet, daß die Zusammensetzung in oral verabreichbarer Form vorliegt.

9. Pharmazeutische Zusammensetzung nach Anspruch 8, dadurch gekennzeichnet, daß die Zusammensetzung eine wässrige Lösung ist, die 1 bis 10 Gew.% des aktiven Behandlungsmittels enthält.

10. Pharmazeutische Zusammensetzung nach einem der Ansprüche 1 bis 7, dadurch gekennzeichnet, daß die Zusammensetzung in injizierbarer Form vorliegt.

11. Pharmazeutische Zusammensetzung nach Anspruch 10, dadurch gekennzeichnet, daß die Zusammensetzung eine injizierbare wässrige Lösung ist, die 1 bis 11 Gew.% des aktiven Behandlungsmittels enthält.

12. Pharmazeutische Zusammensetzung nach einem der vorangehenden Ansprüche, dadurch gekennzeichnet, daß das aktive Behandlungsmittel der einzige pharmakologisch aktive Wirkstoff in der Zusammensetzung ist.

13. Pharmazeutische Zusammensetzung nach einem der Ansprüche 1 bis 11, dadurch gekennzeichnet, daß die Zusammensetzung in Form einer isotonischen wässrigen Lösung eines Zuckers vorliegt, in der ein Alkalimetall- oder Ammoniumbisulfit oder -sulfit gelöst ist, wobei die Lösung eine Gleichgewichtsmischung aus Zucker, Bisulfit oder Sulfit und Bisulfitaddukt des Zuckers enthält.

14. Pharmazeutische Zusammensetzung nach Anspruch 13, dadurch gekennzeichnet, daß sie aus

(A) 4 bis 7 Gew.% mnindestens eines Monosaccharids ausgewählt aus Aldosen und 2-Carbonylketosen mit 5 bis 7 Kohlenstoffatomen,

(B) 1 bis 4 Gew.% eines oder mehrerer Alkalimetall- oder Ammoniumbisulfite oder -sulfite und

(C) 89 bis 95 Gew.% Wasser besteht.

15. Verbindung der Definition gemäß einem der Ansprüche 1 bis 7 sur Verwendung bei der Behandlung einer akuten oder beginnenden Thrombose beim Menschen oder anderen Säugern.

**Patentansprüche für Vertragsstaat: AT**

1. Verfahren zur Herstellung einer pharmazeutischen Zusammensetzung zur Behandlung einer akuten oder beginnenden Thrombose bei Säugern, dadurch gekennzeichnet, daß man mindestens eine Trägersubstanz mit mindestens einer organischen Verbindung als aktivem Behandlungsmittel vermischt, welche die Formel

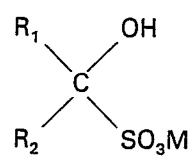

besitzt, in der $R_1$ ein geradkettiger, hydroxysubstituierter Alkylrest mit 3,4 oder 5 hydroxylierten Kohlenstoffatomen ist, $R_2$ ein Wasserstoffatom oder ein Hydroxymethylrest ist und M ein Alkalimetall oder eine Ammoniumgruppe ist.

2. Verfahren nach Anspruch 2, dadurch gekennzeichnet, daß die Ketose Fructose ist.
mittel ein Bisulfitaddukt einer Aldose mit 5 bis 7 Kohlenstoffatomen ist.

3. Verfahren nach Anspruch 2, dadurch gekennzeichnet, daß die Aldose, Arabinose oder Glucose ist.

4. Verfahren nach Anspruch 3, dadurch gekennzeichnet, daß das aktive Behandlungsmittel Natriumglucosebisulfit ist.

5. Verfahren nach Anspruch 1, dadurch gekennzeichnet, daß das aktive Behandlungsmittel ein Bisulfitaddukt einer 2-Carbonylketose mit 5 bis 7 Kohlenstoffatomen ist.

6. Verfahren nach Anspruch 5, dadurch gekennzeichnet, daß die Ketose Fructose ist.

7. Verfahren nach einem der vorangehenden Ansprüche, dadurch gekennzeichnet, daß M Natrium ist.

8. Verfahren nach einem der vorangehenden Ansprüche, dadurch gekennzeichnet, daß die Zusammensetzung in einer oral verabreichbaren Form vorliegt.

9. Verfahren nach Anspruch 8, dadurch gekennzeichnet, daß die Zusammensetzung eine wässrige Lösung ist, die 1 bis 10 Gew.% aktives Behandlungsmittel enthält.

10. Verfahren nach einem der Ansprüche 1 bis 7, dadurch gekennzeichnet, daß die Zusammensetzung in injizierbarer Form vorliegt.

11. Verfahren nach Anspruch 10, dadurch gekennzeichnet, daß die Zusammensetzung eine injizierbare wässrige Lösung ist, die 1 bis 11 Gew.% des aktiven Behandlungsmittels enthält.

12. Verfahren nach einem der vorangehenden Ansprüche, dadurch gekennzeichnet, daß das aktive Behandlungsmittelder einzige pharmakologisch aktive Wirkstoff in der Zusammensetzung ist.

13. Verfahren nach einem der Ansprüche 1 bis 11, dadurch gekennzeichnet, daß die Zusammenseztung in Form einer isotonischen wässrigen Lösung eines Zuckers vorliegt, in der ein Alkalimetall- oder Ammoniumbisulfit oder -sulfit gelöst ist, wobei die Lösung eine Gleichgewichtsmischung aus Zucker, Bisulfit oder Sulfit und Bisulfitaddukt eines Zuckers enthält.

14. Verfahren nach Anspruch 13, dadurch gekennzeichnet, daß die Zusammensetzung aus

(A) 4 bis 7 Gew.% mindestens eines Monosaccharids ausgewählt aus Aldosen und 2-Carbonylketosen mit 5 bis 7 Kohlenstoffatomen,

(B) 1 bis 4 Gew.% eines oder mehrerer Alkalimetall- oder Ammoniumbisulfite oder -sulfite und

(C) 89 bis 95 Gew.% Wasser besteht.